(19) Europäisches Patentamt
European Patent Office
Office européen des brevets

(11) EP 0 630 265 B1

# (12) EUROPEAN PATENT SPECIFICATION

(45) Date of publication and mention of the grant of the patent:
**16.04.2003 Bulletin 2003/16**

(21) Application number: **93911556.4**

(22) Date of filing: **12.03.1993**

(51) Int Cl.⁷: **A61K 51/00**, A61K 51/08, A61K 103/10

(86) International application number:
**PCT/US93/02320**

(87) International publication number:
**WO 93/017719 (16.09.1993 Gazette 1993/22)**

## (54) TECHNETIUM-99m LABELED PEPTIDES FOR IMAGING INFLAMMATION

TECHNETIUM-99-MARKIERTE PEPTIDE ZUR VISUALISIERUNG VOM ENTZUENDUNGEN

PEPTIDES MARQUES AU TECHNETIUM-99m POUR LA MISE EN IMAGE D'UNE INFLAMMATION

(84) Designated Contracting States:
**AT BE CH DE DK ES FR GB GR IE IT LI LU MC NL PT SE**

(30) Priority: **13.03.1992 US 851074**

(43) Date of publication of application:
**28.12.1994 Bulletin 1994/52**

(73) Proprietor: **Diatide, Inc.**
**Londonderry, NH 03053 (US)**

(72) Inventors:
- **DEAN, Richard, T.**
  **Bedford, NH 03110 (US)**
- **LEES, Robert, S.**
  **Brookline, MA 02146 (US)**
- **BUTTRAM, Scott**
  **Derry, NH 03038 (US)**
- **LISTER-JAMES, John**
  **Bedford, NH 03110 (US)**

(74) Representative: **Dörries, Hans Ulrich, Dr. et al**
**Dörries Frank-Molnia Pohlman**
**Postfach 22 16 61**
**80506 München (DE)**

(56) References cited:
**EP-A- 0 174 853**
**EP-A- 0 398 143**
**EP-A- 0 403 243**
**WO-A-89/10759**
**WO-A-90/10463**
**WO-A-91/16919**
**WO-A-92/13572**

- **INORGANIC CHEMISTRY vol. 29, no. 16, 1990, pages 2948 - 2951 N. BRYSON ET AL. 'PROTECTING GROUPS IN THE PREPARATION OF THIOLATE COMPLEXES OF TECHNETIUM' cited in the application**
- **BIOCONJUGATE CHEMISTRY vol. 1, no. 1, 1990, pages 132 - 137 K.E. BAIDOO ET AL. 'SYNTHESIS OF A DIAMINEDITHIOL BIFUNCTIONAL CHELATING AGENT FOR INCORPORATION OF TECHNETIUM-99M INTO BIOMOLECULES' cited in the application**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

## BACKGROUND OF THE INVENTION

### 1. Field of the Invention

This invention relates to radiodiagnostic agents and reagents for preparing such agents, and also methods for producing radiolabeled radiodiagnostic agents. Specifically, the invention relates to technetium-99m (Tc-99m) labeled agents, methods and kits for making such agents, and use of such agents to image sites of infection and inflammation in a mammalian body.

### 2. Description of the Prior Art

A variety of radionuclides are known to be useful for radioimaging, including $^{67}$Ga, $^{99m}$Tc (Tc-99m), $^{111}$In, $^{123}$I, $^{125}$I, $^{169}$Yb or $^{186}$Re. The sensitivity of imaging methods using radioactively-labeled peptides is much higher than other techniques known in the art, since the specific binding of the radioactive peptide concentrates the radioactive signal over the area of interest, for example, an inflammatory site.

There is a clinical need to be able to determine the location and/or extent of sites of focal or localized infection. In a substantial number of cases conventional methods of diagnosis (such as physical examination, x-ray, CT and ultrasonography) fail to identify such sites (*e.g.*, an abscess). In some cases, biopsy may be resorted to, but is preferably avoided at least until it is necessary in order to identify the pathogen responsible for an abscess at a known location. Identifying the site of such "occult" infection is important because rapid localization of the problem is critical to effective therapeutic intervention.

In the field of nuclear medicine, certain pathological conditions can be localized or the extent of such conditions determined by imaging the internal distribution of administered radioactively-labeled tracer compounds (i.e. radiotracers or radiopharmaceuticals) that accumulate specifically at the pathological site. However, an abscess may be caused by any one of many possible pathogens, so that a radiotracer specific for a particular pathogen would have limited scope. On the other hand, infection is almost invariably accompanied by inflammation, which is a general response of the body to tissue injury. Therefore, a radiotracer specific for sites of inflammation would be expected to be useful in localizing sites of infection caused by any pathogen.

One of the main phenomena associated with inflammation is the localization of leukocytes (white blood cells), usually monocytes and neutrophils, at the site of inflammation. A radiotracer specific for leukocytes would be useful in detecting leukocytes at the site of a localized infection. Currently approved nuclear medicine procedures for imaging sites of infection use either indium-111 labeled leukocytes ($^{111}$In-WBC) (*see, e.g.* Peters, 1992, J. Nucl. Med. 33: 65-67) or gallium-67 ($^{67}$Ga) citrate *(see, e.g.* Ebright *et al*., 1982, Arch. Int. Med. 142: 246-254).

A major disadvantage of using $^{111}$In-labeled WBCs is that the preparation of the radiotracer requires sterile removal of autologous blood, sterile isolation of the leukocytes from the blood, sterile labeling of the leukocytes using conditions that do not damage the cells (since damaged WBC are taken up by the reticuloendothelial system when re-injected) and return (re-injection) of the (now labeled) leukocytes to the patient. Furthermore, a delay of 12 to 48 hours between injection and imaging may be required for optimal images. While Tc-99m labeled leukocytes have been used to shorten this delay period *(see, e.g.* Vome *et al*., 1989, J. Nucl. Med. 30: 1332-1336), ex-corporeal labeling is still required. A preferred radiotracer would be one that does not require removal and manipulation of autologous blood components.

$^{67}$Ga-citrate can be administered by intravenous injection. However, this compound is not specific for sites of infection or inflammation. Moreover, a delay of up to 72 hours is often required between injection of the radiotracer and imaging. In addition, the $\gamma$-(gamma) emission energies of $^{67}$Ga are not well suited to conventional gamma cameras.

Radiolabeled monoclonal and polyclonal antibodies raised against human leukocytes (including monocytes, neutrophils, granulocytes and other) have been developed. Tc-99m labeled antigranulocyte monoclonal antibodies *(see, e.g.* Lind *et al*., 1990, J. Nucl. Med. 31: 417-473) and $^{111}$In-labeled non-specific human immunoglobulin (*see*, *e.g.* LaMuraglia *et al*., 1989, J. Vasc. Surg. 10: 20-28) have been tested for the detection of inflammation secondary to infection. $^{111}$In-labeled IgG shares the disadvantages of $^{111}$In-labeled WBC, in that 24-48 hours are required between injection and optimal imaging. In addition, all radiolabeled antibodies are difficult to produce and face protracted regulatory agency approval procedures as biologics.

Small readily synthesized molecules are preferred for routinely used radiopharmaceuticals. There is clearly a need for small synthetic molecules that can be directly injected into a patient and will image sites of infection and inflammation by localizing at sites where leukocytes have accumulated.

One class of compounds known to bind to leukocytes are chemotactic peptides that cause leukocytes to move up a peptide concentration gradient *(see* Wilkinson, 1988, Meth. Enzymol. 162: 127-132). These compounds bind to

receptors on the surface of leukocytes with very high affinity. These peptides are derived from a number of sources, including complement factors, bacteria, tuftsin, elastin, fibrinopeptide B, fibrinogen Bβ, platelet factor 4 and others. Small synthetic peptides derived from these chemotactic compounds and radiolabeled would be very useful as radiotracers for imaging sites of inflammation *in vivo*.

Radiolabeled peptides have been reported in the prior art.

U.S. Patent No. 4,986,979 relates to the use of radiolabeled chemotactic formyl peptides to radiolabel leukocytes ex-corporeally *via* a photoaffinity label.

EPC 90108734.6 relates to chemotactic formyl peptide - $^{111}$In-labeled DTPA conjugates.

PCT W090/10463 relates to the use of radiolabeled chemotactic formyl peptides to radiolabel leukocytes ex-corporeally *via* a photoaffinity label.

Zoghbi *et al*., 1981, J. Nucl. Med. 22: 32 (Abst) disclose formyl peptide chemotactic factors derived from bacteria coupled to $^{111}$In-labeled transferrin.

Jiang *et al*., 1982, Nuklearmedizin 21: 110-113 disclose a chemotactic formylated peptide radiolabeled with $^{125}$I.

Fischman *et al.,* 1991, J. Nucl. Med. 32: 482-491 relates to chemotactic formyl peptide - $^{111}$In-labeled DTPA conjugates.

The use of chelating agents for radiolabeling polypeptides, methods for labeling peptides and polypeptides with Tc-99m are known in the prior art and are disclosed in co-pending U.S. Patent Applications Serial Nos. 07/653,012, 07/807,062, 07/871,282, and 07/893,981, which are hereby incorporated by reference.

## SUMMARY OF THE INVENTION

The present invention provides scintigraphic imaging agents that are peptide reagents radioactively-labeled with Tc-99m. The peptide reagents of the invention are comprised of specific binding peptides that bind leukocytes, covalently linked to a Tc-99m radiolabel binding moiety.

A first aspect of the invention comprises reagents for preparing scintigraphic imaging agents for imaging sites of inflammation within a mammalian body, said reagents comprising a leukocyte binding peptide having an amino acid sequence comprising between 3 and 100 amino acids and a Tc-99m radiolabel-binding moiety.

The radiolabeled peptide reagents of the invention comprise a specific binding peptide that binds to leukocytes, and a Tc-99m radiolabel-binding moiety of formula

I.

Cp(aa)Cp

wherein Cp is a protected cysteine residue and (aa) stands for an amino acid, and wherein the radiolabel-binding moiety is covalently linked to the specific binding peptide. In a preferred embodiment, the amino acid is glycine. In another preferred embodiment, the radiolabel-binding moiety is linked to the specific peptide *via* one or more amino acids.

In another aspect, the invention provides peptide reagents comprising a Tc-99m radiolabel-binding moiety having the following structure:

II.

$$A\text{-}CZ(B)\text{-}[C(R^1R^2)]_n\text{-}X$$

wherein A is H, HOOC, $H_2NOC$, (peptide)-NHOC, (peptide)-OOC or $R^4$; B is H, SH or $-NHR^3$, $-N(R^3)$-(peptide) or $R^4$; Z is H or $R^4$; X is H, SH or $-NHR^3$, $-N(R^3)$-(peptide) or $R^4$; $R^1$, $R^2$, $R^3$ and $R^4$ are independently H or straight or branched chain or cyclic lower alkyl; n is 0, 1 or 2; and: (1) where B is - $NHR^3$ or $-N(R^3)$-(peptide), X is SH and n is 1 or 2; (2) where X is $-NHR^3$ or $-N(R^3)$-(peptide), B is SH and n is 1 or 2; (3) where B is H or $R^4$, A is HOOC, $H_2NOC$, (peptide) -NHOC or (peptide)-OOC, X is SH and n is 0 or 1; (4) where A is H or $R^4$, then where B is SH, X is $-NHR^3$ or $-N(R^3)$ -(peptide) and where X is SH, B is $-NHR^3$ or $-N(R^3)$-(peptide); (5) where X is H or $R^4$, A is HOOC, $H_2NOC$, (peptide) -NHOC or (peptide)-OOC and B is SH; (6) where Z is methyl, X is methyl, A is HOOC, $H_2NOC$, (peptide)-NHOC or (peptide)-OOC and B is SH and n is 0; and (7) where B is SH and X is SH, n is not 0; and wherein the thiol moiety is in the reduced form.

Another aspect of the invention provides reagents for preparing scintigraphic imaging agents for imaging sites

of inflammation within a mammalian body, the reagents comprising a leukocyte-binding peptide having an amino acid sequence comprising between 3 and 100 amino acids and a Tc-99m radiolabel-binding moiety that forms a Tc-99m complex that is electrochemically neutral.

In yet another aspect, the present invention provides reagents comprising leukocyte-binding peptides covalently linked to a Tc-99m radiolabel-binding moiety having the following structure:

III.

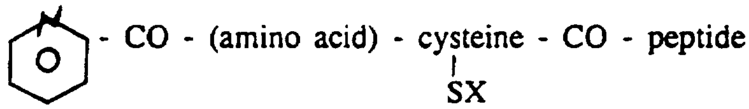

[for purposes of this invention, radiolabel-binding moieties having this structure will be referred to as picolinic acid (Pic)-based moieties];
or

IV.

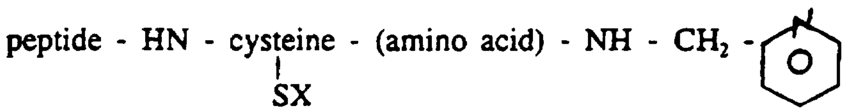

[for purposes of this invention, radiolabel-binding moieties having this structure will be referred to as picolylamine (Pica)-based moieties]; wherein X is H or a protecting group; (amino acid) is any amino acid; the Tc-99m radiolabel-binding moiety is covalently linked to the peptide, and the complex of the radiolabel-binding moiety and Tc-99m is electrically neutral. In a preferred embodiment, the amino acid is glycine and X is an acetamidomethyl protecting group. In additional preferred embodiments, the peptide is covalently linked to the Tc-99m radiolabel-binding moiety via an amino acid, most preferably glycine.

In yet another embodiment of the invention, reagents are provided for preparing scintigraphic imaging agents for imaging sites within a mammalian body, comprising a specific binding peptide and a bisamino bisthiol Tc-99m radiolabel-binding moiety covalendy linked to the peptide. The bisamino bisthiol Tc-99m radiolabel-binding moiety in this embodiment of the invention has a formula selected from the group consisting of:

V.

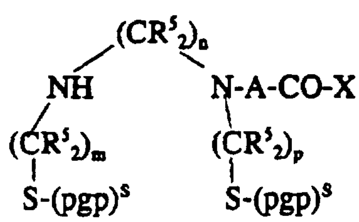

wherein each $R^5$ can be independently H, $CH_3$ or $C_2H_5$; each $(pgp)^s$ can be independently a thiol protecting group or H; m, n and p are independently 2 or 3; A is linear or cyclic lower alkyl, aryl, heterocyclyl, combinations or substituted derivatives thereof; and X is a peptide;

VI.

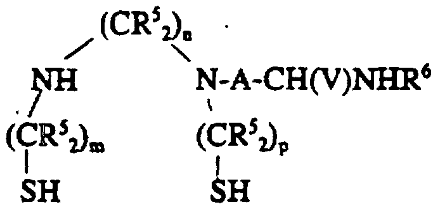

wherein each $R^5$ is independently H, lower alkyl having 1 to 6 carbon atoms, phenyl, or phenyl substituted with lower alkyl or lower alkoxy; m, n and p are independently 1 or 2; A is linear or cyclic lower alkyl, aryl, heterocyclyl, combinations or substituted derivatives thereof; V is H or CO-peptide; $R^6$ is H or a peptide; provided that when V is H, $R^6$ is a peptide and when $R^6$ is H, V is a peptide. [For purposes of this invention, radiolabel-binding moieties having these structures will be referred to as "BAT" moieties]. In one preferred embodiment, the peptide is covalently linked to the Tc-99m radiolabel-binding moiety via an amino acid, most preferably glycine.

The specific binding peptides of the invention may also be covalently linked to a polyvalent linking moiety. Polyvalent linking moieties of the invention are comprised of at least 2 identical linker functional groups capable of covalently bonding to specific binding peptides or Tc-99m binding moieties. Preferred linker functional groups are primary or secondary amines, hydroxyl groups, carboxylic acid groups or thiol-reactive groups. In preferred embodiments, the polyvalent linking moieties are comprised of *bis*-succinimdylmethylether (BSME), 4-(2,2-dimethylacetyl)benzoic acid (DMAB) and *tris*(succinimidylethyl)amine (TSEA).

The invention comprises scintigraphic imaging agents that are complexes between the reagents of the invention and Tc-99m, and methods for radiolabeling the reagents of the invention with Tc-99m. Radiolabeled complexes provided by the invention are formed by reacting the reagents of the invention with Tc-99m in the presence of a reducing agent. Preferred reducing agents include but are not limited to dithionite ion, stannous ion and ferrous ion. Complexes of the invention are also formed by labeling the reagents of the invention with Tc-99m by ligand exchange of a prereduced Tc-99m complex as provided herein.

The invention also provides kits for preparing scintigraphic imaging agents that are the reagents of the invention radiolabeled with Tc-99m. Kits for labeling the reagents provided by the invention with Tc-99m are comprised of a sealed vial containing a predetermined quantity of a reagent of the invention and a sufficient amount of reducing agent to label the reagent with Tc-99m.

This invention provides methods for preparing peptide reagents of the invention by chemical synthesis *in vitro.* In a preferred embodiment, peptides are synthesized by solid phase peptide synthesis.

This invention provides the use of scintigraphic imaging agents that are Tc-99m labeled reagents for imaging sites of inflammation within a mammalian body by obtaining *in vivo* gamma scintigraphic images. These methods comprise administering an effective diagnostic amount of Tc-99m labeled reagents of the invention and detecting the gamma radiation emitted by the Tc-99m label localized at the site of inflammation within the mammalian body.

Specific preferred embodiments of the present invention will become evident from the following more detailed description of certain preferred embodiments and the claims.

## BRIEF DESCRIPTION OF THE DRAWINGS

Figure 1 illustrates a gamma-scintiphoto of a New Zealand white rabbit treated as described in Example 3.

## DETAILED DESCRIPTION OF THE INVENTION

The present invention provides reagents for preparing Tc-99m radiolabeled scintigraphic imaging agents for imaging target sites within a mammalian body. The reagents comprise a specific binding peptide that binds to leukocytes, covalently linked to a Tc-99m radiolabel complexing group.

The peptides of this invention bind to leukocytes, preferably monocytes and neutrophils and most preferably to neutrophils. For purposes of this invention, the term "bind to leukocytes" is intended to mean that the peptides of the present invention are capable of accumulating at sites of infection or inflammation in mammalian body sufficient to allow detection of such sites by gamma scintigraphy.

In Cp(aa)Cp-containing peptides, the Cp is a protected cysteine where the S-protecting groups are the same

or different and may be but not limited to:

- $CH_2$-aryl (aryl is phenyl or alkyl or alkyloxy substituted phenyl);
- CH-$(aryl)_2$, (aryl is phenyl or alkyl or alkyloxy substituted phenyl);
- C-$(aryl)_3$, (aryl is phenyl or alkyl or alkyloxy substituted phenyl);
- $CH_2$-(4-methoxyphenyl);
- CH-(4-pyridyl)$(phenyl)_2$;
- $C(CH_3)_3$
- 9-phenylfluorenyl;
- $CH_2$NHCOR (R is unsubstituted or substituted alkyl or aryl);
- $CH_2$-NHCOOR (R is unsubstituted or substituted alkyl or aryl);
- CONHR (R is unsubstituted or substituted alkyl or aryl);
- $CH_2$-S-$CH_2$-phenyl

The preferred protecting group has the formula -$CH_2$-NHCOR wherein R is a lower alkyl having I and 8 carbon atoms, phenyl or phenyl-substituted with lower alkyl, hydroxyl, lower alkoxy, carboxy, or lower alkoxycarbonyl.

Labeling with Tc-99m is an advantage of the present invention because the nuclear and radioactive properties of this isotope make it an ideal scintigraphic imaging agent. This isotope has a single photon energy of 140 keV and a radioactive half-life of about 6 hours, and is readily available from a $^{99}$Mo-$^{99m}$Tc generator. Other radionuclides known in the prior art have effective half-lives which are much longer (*for example*, $^{111}$In, which has a half-life of 67.4 h) or are toxic (*for example,* $^{125}$I).

Each specific-binding peptide-containing embodiment of the invention is comprised of a sequence of amino acids. Particular amino acids comprising the peptides of this invention may be L- or D- amino acids, naturally occurring and otherwise; D-amino acids are indicated by a subscript D. Reagents provided by the invention include but are not limited to the following compounds:

Leukocyte Binding Peptides

*acetyl*.C$_{Acm}$GC$_{Acm}$.Aca.(VPGVG)$_4$amide

(*acetyl*.CC$_{Acm}$GC$_{Acm}$PLYKKIIKKLLES)$_2$.BSME

*acetyl*.CGGGPLYKKIIKKLLES

Pic.GC(VGVAPG)$_3$amide

*p*Glu.GVNDNEEGFFSARGGCamide

*acetyl*.(LKKL)$_5$C$_{Acm}$GC$_{Acm}$amide

[BAT].GGPLYKKIIKKLLES

*formyl*.MLFK.[BAT].amide

*formyl*.Thp.LF.[BAM]

*formyl*.MLFK.[BAT]

[BAT].(VPGVG)$_4$amide

*formyl*.MLFK.[BAT].KKKKKamide

*formyl*.MLFK.[BAT].GSGSamide

*formyl*.MLFK.[BAT].E

*formyl*.MLFC$_{Acm}$GC$_{Acm}$

C$_{Acm}$GC$_{Acm}$(VGVAPG)$_3$

*formyl*.MILFC$_{Acm}$GC$_{Acm}$

C$_{Acm}$GC$_{Acm}$TKPR

*formyl*.MLFC$_{Acm}$G.Pica

*formyl*.Nle.LF.Nle.YKC$_{Acm}$GC$_{Acm}$

Pic.GC$_{Acm}$(VGVAPG)$_3$amide

Pic.GC$_{Acm}$(VPGVG)$_4$amide

Pic.GC$_{Acm}$PLYKKIIKKLLES

C$_{Acm}$GC$_{Acm}$GGPLYKKIIKKLLES

*p*Glu.GVNDNEEGFFSARC$_{Acm}$GC$_{Acm}$amide

(VPGVG)$_4$GGGC$_{Acm}$GC$_{Acm}$amide

(VGVAPG)$_3$GGGC$_{Acm}$GC$_{Acm}$amide

*acetyl*.C$_{Acm}$GC$_{Acm}$GGG(VPGVG)$_4$amide

**Leukocyte Binding Peptides (cont'd.)**

| | |
|---|---|
| *formyl*.MLFK.[BAT].EGE | *formyl*.MLF(NHCH$_2$CH$_2$OCH$_2$CH$_2$OCH$_2$CH$_2$NH)Pic.GC$_{Mob}$ |
| *formyl*.M.DpgF.[BAM] | (*acetyl*.CKKC$_{Acm}$GC$_{Acm}$PLYKKIIKKLLES)$_2$.BSME |
| [BAT].(VGVAPG)$_3$amide | [BAT].GHRPLDKKREEAPSLRPAPPPISGGGYRamide |
| [DTPA].C$_{Acm}$GC$_{Acm}$PLYKKIIKKLLES | *acetyl*.KKKKKC$_{Acm}$GC$_{Acm}$GGPLYKKIIKKLLES |
| [BAT].KKLLKKLYKKIIKKLLES | (*formyl*.MLFK.[BAT].GGC$_{Acm}$GC$_{Acm}$GGC.amide)$_2$.BSME |
| *acetyl*.CKKC$_{Acm}$GC$_{Acm}$PLYKKIIKKLLES | Pic.GC$_{Acm}$GHRPLDKKREEAPSLRPAPPPISGGGYRamide |
| (*acetyl*CGC$_{Acm}$GC$_{Acm}$GGPLYKKIIKKLLES)$_2$.BSME | (*formyl*MLFKGGC$_{Acm}$GC$_{Acm}$GGCamide)$_2$.BSME |

[Single-letter abbreviations for amino acids can be found in G. Zubay, *Biochemistry* (2d. ed.), 1988 (MacMillen Publishing: New York) p.33; Dgp = dipropylglycine; *p*Glu = *pyro*-glutamic acid; Nle = norleucine; Acm = acetamidomethyl; Mob = 4-Methoxybenzyl; Aca = ε-aminocaproic acid; Pic = picolinic acid; Pica = picolylamine (2-(aminomethyl)pyridine); [BAT] = $N^6$,$N^9$-*bis*(2-methyl-2-mercaptopropyl)-6,9-diazanonanoic acid; Thp = 4-aminotetrahydrothiopyran-4-carboxylic acid; [BAM] = $N^6$,$N^9$-*bis*(2-methyl-2-mercaptopropyl)-1,6,9-triazanonanoic acid; BSME = *bis*-succinimidylmethylether; DTPA = diethylenetriamine pentaacetic acid; peptides are linked to BSME linkers *via* the free thiol moiety of the unprotected cysteine residue (C) in each such peptide).

Peptides of the present invention can be chemically synthesized *in vitro*. Peptides of the present invention

can generally advantageously be prepared on an amino acid synthesizer. The peptides of this invention can be synthesized wherein the complexing group is covalently linked to the peptide during chemical *in vitro* synthesis, using techniques well known to those with skill in the art. Such peptides covalently-linked to the complexing group upon synthesis are advantageous because specific sites of covalent linkage can be determined therein.

Radiolabel binding moieties of the invention may be introduced into the target specific peptide during peptide synthesis. For embodiments comprising picolinic acid [(Pic-); *e.g.*, Pic-Gly-Cys(protecting group)-], the radiolabel-binding moiety can be synthesized as the last (i.e., ammo-terminal) residue in the synthesis. In addition, the picolinic acid-containing radiolabel-binding moiety may be covalently linked to the ∈-amino group of lysine to give, for example, αN (Fmoc)-Lys-∈N[Pic-Gly-Cys(protecting group)], which may be incorporated at any position in the peptide chain. This sequence is particularly advantageous as it affords an easy mode of incorporation into the target binding peptide.

Similarly, the picolylamine (Pica)-containing radiolabel-binding moiety [-Cys(protecting group)-Gly-Pica] can be prepared during peptide synthesis by including the sequence [-Cys(protecting group)-Gly-] at the carboxyl terminus of the peptide chain. Following cleavage of the peptide from the resin the carboxyl terminus of the peptide is activated and coupled to picolylamine. This synthetic route requires that reactive side-chain functionalities remain masked (protected) and do not react during the conjugation of the picolylamine.

Examples of small synthetic peptides containing the Pic-Gly-Cys- and - Cys-Gly-Pica chelators are provided in the Examples hereinbelow. This invention provides for the incorporation of these chelators into virtually any peptide capable of specifically binding to leukocytes *in vivo*, resulting in a radiolabeled peptide having Tc-99m held as neutral complex.

This invention also provides specific-binding small synthetic peptides which incorporate bisamine bisthiol (BAT or BAM) chelators which may be labeled with Tc-99m. This invention provides for the incorporation of these chelators into virtually any peptide capable of specifically binding to leukocytes *in vivo*, resulting in a radiolabeled peptide having Tc-99m held as neutral complex. An examples of a small synthetic peptide containing a BAT chelator as radiolabel-binding moiety is provided in the Examples hereinbelow.

The specific binding peptides of the invention may also be covalently linked to a polyvalent linking moiety. Polyvalent linking moieties provided by the invention are comprised of at least 2 linker functional groups capable of covalently bonding to platelet-specific moieties, including linear and cyclic peptides. Such functional groups include but are not limited to primary and secondary amines, hydroxyl groups, carboxylic acid groups and thiol reactive groups. Polyvalent linking moieties are comprised of preferably at least three functional groups capable of being covalently linked to platelet-specific moieties, including linear and cyclic peptides. Preferred polyvalent linking moieties include amino acids such as lysine, homolysine, ornithine, aspartic acid and glutamic acid; linear and cyclic amines and polyamines; polycarboxylic acids; activated thiols; and thiol-reactive reagents such as di- and tri-maleimides. Also preferred are embodiments wherein the polyvalent linking moieties comprise a multiplicity of polyvalent linking moieties covalently linked to form a branched polyvalent linking moiety. Most preferred polyvalent linking moieties include *bis*-succinimidylmethylether, *tris*(succinimidylethyl)amine, 4-(2,2-dimethylacetyl)benzoic acid (DMAB) and derivatives thereof.

In forming a complex of radioactive technetium with the reagents of this invention, the technetium complex, preferably a salt of Tc-99m pertechnetate, is reacted with the reagent in the presence of a reducing agent. Preferred reducing agents are dithionite, stannous and ferrous ions; the most preferred reducing agent is stannous chloride. Means for preparing such complexes are conveniently provided in a kit form comprising a sealed vial containing a predetermined quantity of a reagent of the invention to be labeled and a sufficient amount of reducing agent to label the reagent with Tc-99m. Alternatively, the complex may be formed by reacting a reagent of this invention with a preformed labile complex of technetium and another compound known as a transfer ligand. This process is known as ligand exchange and is well known to those skilled in the art. The labile complex may be formed using such transfer ligands as tartrate, citrate, gluconate or mannitol, for example. Among the Tc-99m pertechnetate salts useful with the present invention are included the alkali metal salts such as the sodium salt, or ammonium salts or lower alkyl ammonium salts.

The reaction of the peptides of this invention with Tc-pertechnetate or preformed Tc-99m labile complex can be carried out in an aqueous medium at room temperature. When an anionic complex having a charge of [-1] is formed in the aqueous medium in the form of a salt with a suitable cation such as sodium cation, ammonium cation, mono, di- or tri-lower alkyl amine cation, etc. Any conventional salt of the anionic complex with a pharmaceutically acceptable cation can be used in accordance with this invention.

In a preferred embodiment of the invention, a kit for preparing technetium-labeled peptides is provided. The peptides of the invention can be chemically synthesized using methods and means well-known to those with skill in the art and described hereinbelow. Peptides thus prepared are comprised of between 3 and 100 amino acid residues, and are covalently linked to a radioisotope complexing group wherein the complexing group binds a radioisotope. An appropriate amount of the peptide is introduced into a vial containing a reducing agent, such as stannous chloride, in an amount sufficient to label the peptide with Tc-99m. An appropriate amount of a transfer ligand as described (such

as tartrate, citrate, gluconate or mannitol, for example) can also be included. Technetium-labeled peptides according to the present invention can be prepared by the addition of an appropriate amount of Tc-99m or Tc-99m complex into the vials and reaction under conditions described in Example 2 hereinbelow.

Radioactively labeled peptides provided by the present invention are provided having a suitable amount of radioactivity. In forming the Tc-99m radioactive anionic complexes, it is generally preferred to form radioactive complexes in solutions containing radioactivity at concentrations of from about 0.01 millicurie (mCi) to 100 mCi per ml.

Technetium-labeled peptides provided by the present invention can be used for visualizing sites of inflammation, including abscesses and sites of "occult" infection. The Tc-99m labeled peptides provided by the present invention can also be used for visualizing sites of inflammation caused by tissue ischemia, including such disorders as inflammatory bowel disease and arthritis. In accordance with this invention, the technetium-labeled peptides or anionic complexes either as a complex or as a salt with a pharmaceutically acceptable cation are administered in a single unit injectable dose. Any of the common carriers known to those with skill in the art, such as sterile saline solution or plasma, can be utilized after radiolabeling for preparing the injectable solution to diagnostically image various organs, tumors and the like in accordance with this invention. Generally, the unit dose to be administered has a radioactivity of about 0.01 mCi to about 100 mCi, preferably 1 mCi to 20 mCi. The solution to be injected at unit dosage is from about 0.01 ml to about 10 ml. After intravenous administration, imaging of the organ or tumor *in vivo* can take place in a matter of a few minutes. However, imaging can take place, if desired, in hours or even longer, after injecting into patients. In most instances, a sufficient amount of the administered dose will accumulate in the area to be imaged within about 0.1 of an hour to permit the taking of scintiphotos. Any conventional method of scintigraphic imaging for diagnostic purposes can be utilized in accordance with this invention.

The technetium-labeled peptides and complexes provided by the invention may be administered intravenously in any conventional medium for intravenous injection such as an aqueous saline medium, or in blood plasma medium. Such medium may also contain conventional pharmaceutical adjunct materials such as, for example, pharmaceutically acceptable salts to adjust the osmotic pressure, buffers, preservatives and the like. Among the preferred media are normal saline and plasma.

The methods for making and labeling these compounds are more fully illustrated in the following Examples. These Examples illustrate certain aspects of the above-described method and advantageous results. These Examples are shown by way of illustration and not by way of limitation.

## EXAMPLE 1

### Solid Phase Peptide Synthesis

Solid phase peptide synthesis (SPPS) was carried out on a 0.25 millimole (mmole) scale using an Applied Biosystems Model 431A Peptide Synthesizer and using 9-fluorenylmethyloxycarbonyl (Fmoc) amino-terminus protection, coupling with dicyclohexylcarbodiimide/hydroxybenzotriazole or 2-(1H-benzo-triazol-1-yl)-1,1,3,3-tetramethyluronium hexafluorophosphate/-hydroxybenzotriazole (HBTU/HOBT), and using *p*-hydroxymethylphenoxymethylpolystyrene (HMP) resin for carboxyl-terminus acids or Rink amide resin for carboxyl-terminus amides. Resin-bound products were routinely cleaved using a solution comprised of trifluoroacetic acid, water, thioanisole, ethanedithiol, and triethylsilane, prepared in ratios of 100 : 5 : 5 : 2.5 : 2 for 1.5 - 3 h at room temperature. Where appropriate, N-α-formyl groups were introduced either by treating the free N-terminus of a peptide bound to the resin with formic anhydride in dichloromethane at 0°C for 2h or by treating the cleaved, deprotected peptide with acetic anhydride in 98% formic acid. Where appropriate, N-α-acetyl groups were introduced by treating the free N-terminal amino group of the peptide bound to the resin with 20% (v/v) acetic anhydride in NMP for 30 min. Where appropriate the "Pica'" group was introduced by conjugating picolylamine to a precursor peptide using diisopropylcarbodiimide and N-hydroxysuccimmide. Where appropriate, N-terminal [BAT] groups were introduced by treating free N-termina amino groups of the peptide with $N^6$,$N^9$-*bis*(2-methyl-2-triphenylmethylthiopropyl)-$N^6$-(*t*-butoxycarbonyl)-6,9-diazanonanoic acid *N*-hydroxysuccinimide ester.

Where appropriate, BSME adducts were prepared by reacting single thiol-containing peptides (5 to 50 mg/mL in 50 mM sodium phosphate buffer, 4 pH 8) with 0.5 molar equivalents of BMME (*bis*-maleimidomethylether) predissolved in acetonitrile at room temperature for approximately 1 to 18 hours. The solution was concentrated and the product was purified by HPLC.

Crude peptides were purified by preparative high pressure liquid chromatography (HPLC) using a Waters Delta Pak C18 column and gradient elution using 0.1% trifluoroacetic acid (TFA) in water modified with acetonitrile. Acetonitrile was evaporated from the eluted fractions which were then lyophilized. The identity of each product was confirmed by fast atom bombardment mass spectroscopy (FABMS) or by electrospray mass spectroscopy (ESMS).

**EXAMPLE 2**

**A General Method for Radiolabeling with Tc-99m**

0.1 mg of a peptide prepared as in Example 1 was dissolved in 0.1 mL of 0.05M potassium phosphate buffer (pH 7.4). Tc-99m gluceptate was prepared by reconstituting a Glucoscan vial (E.I. DuPont de Nemours, Inc.) with 1.0 mL of Tc-99m sodium pertechnetate containing up to 200 mCi and allowed to stand for 15 minutes at room temperature. 25 μl of Tc-99m gluceptate was then added to the peptide and the reaction allowed to proceed at room temperature or at 100°C for 15 to 30 min and then filtered through a 0.2 μm filter.

The Tc-99m labeled peptide purity was determined by HPLC using a Vydak 218TP54 (RP-18, 5 micron, 220 x 4.6 mm) or Waters DeltaPak (RP-18, 5 micron, 150 x 3.9 mm) analytical column and eluted as described in the Footnotes in Table I. Radioactive components were detected by an in-line radiometric detector linked to an integrating recorder. Tc-99m gluceptate and Tc-99m sodium pertechnetate elute between 1 and 4 minutes under these conditions, whereas the Tc-99m labeled peptide eluted after a much greater amount of time.

The following Table illustrates successful Tc-99m labeling of peptides prepared according to Example 1 using the method described herein. Particular applications of the method are as follows:

HPLC methods (indicated by superscript after $R_t$ in the Table below):

| | | |
|---|---|---|
| Method 1 | Brownlee column | 100% A to 100% $B_{70}$ in 10 min |
| Method 2 | Vydak column | 100% A to 100% $B_{90}$ in 10 min |
| Method 3 | Vydak column | 100% A to 100% $B_{70}$ in 10 min |
| Method 4 | Waters column | 100% A to 100% $B_{90}$ in 10 min |
| Method 5 | Waters column | 100% A to 100% $B_{90}$ in 20 min |

wherein:

solvent A = 0.1 % $CF_3COOH/H_2O$
solvent $B_{70}$ = 0.1% $CF_3COOH$/70% $CH_3CN/H_2O$
solvent $B_{90}$ = 0.1% $CF_3COOH$/90% $CH_3CN/H_2O$
solvent flow rate = 1 mL/min

Vydak column = 218TP54 RP-18, 5μ, 220mm x 4.6mm analytical column Brownlee column = Spheri-5, RP-18 5μ, 220 x 4.6mm column Waters column = DeltaPak RP-18, 5μ, 150mm x 3.9mm analytical column

| Peptide Reagents | FABMS MH$^+$ | Radiochemical Yield | HPLC $R_t$(min) |
|---|---|---|---|
| $C_{Mob}GC_{Acm}$PLYKKIIKKLLES | 2028 | 97% | Bound |
| *formyl*.MLF$C_{Acm}GC_{Acm}$ | 843 | 100% | 11.1,11.9[1] |
| $C_{Acm}GC_{Acm}$(VGVAPG)$_3$amide | 1865 | 100% | 17.7[1] |
| *formyl*.MIFL$C_{Acm}GC_{Acm}$ | 957 | 100% | 11.4[1] |
| $C_{Acm}GC_{Acm}$TKPR | 906.5 | 100% | 16.1[1] |
| *formyl*.MLF$C_{Acm}$G.Pica | 760 | 100% | 10.9,12.2[1] |
| *formyl*.Nle.LF.Nle.YK$C_{Acm}GC_{Acm}$ | 1230 | 97% | 15.6-16.8[2] |
| Pic.G$C_{Acm}$(VGVAPG)$_3$amide | 1795 | 92% | 12.4[2] |
| Pic.G$C_{Acm}$(VPGVG)$_4$amide | 1992 | 100% | 12.0[1] |
| Pic.G$C_{Acm}$PLYKKIIKKLLES | 1910 | 81% | 12.9,13.3[3] |
| $C_{Acm}GC_{Acm}$GGPLYKKIIKKLLES | 2093 | 96% | 12.6[3] |
| *p*Glu.GVNDNEEGFFSAR$C_{Acm}GC_{Acm}$amide | 1957 | 95% | 16.3,16.7[3] |
| PicG$C_{Acm}$GHRPLDKKREEAPSLRPAPPPISGGYR | 3377 | 94% | 11.3[3] |
| (VPGVG)$_4$GGG$C_{Acm}GC_{Acm}$amide | 2231 | 67% | 11.2,11.5[3] |
| (VGVAPG)$_3$GGG$C_{Acm}GC_{Acm}$amide | 2035 | 33% | 10.6[3] |
| *Ac*.$C_{Acm}GC_{Acm}$GGG(VPGVG)$_4$amide | 2275 | 97% | 9.6,9.9[3] |
| *Ac*.$C_{Acm}GC_{Acm}$.Aca.(VPGVG)$_4$amide | 2216 | 76% | 11.6,12.3[3] |

| Peptide Reagents | FABMS $MH^+$ | Radiochemical Yield | HPLC $R_t$(min) |
|---|---|---|---|
| *Ac*.CGGGPLYKKIIKKLLES | 1889 | 83% | 14.1-21.8[2] |
| Pic.GC(VGVAPG)$_3$amide | 1724 | 100% | 17.4[2] |
| *p*Glu.GVNDNEEGFFSARGGCamide | 1768* | 94% | 16.6,12.4[2] |
| *Ac*.(LKKL)$_5$C$_{Acm}$GC$_{Acm}$amide | 2878 | 63% | 17.0-18.0[2] |
| [BAT]GGPLYKKIIKKLLES | 2006 | 94% | 9.5[4] |
| [BAT]GHRPLDKKREEAPSLRPAPPPISGGGYRamide | 3357 | 93% | 10.4,11.6[2] |
| *formyl*.MLFK.[BAT].amide | 884 | 99% | 12.6[2] |
| *formyl*.Thp.LF.[BAM] | 775** | 99% | 13.3,13.6[2] |
| *Ac*.KKKKKC$_{Acm}$GC$_{Acm}$GGPLYKKIIKKLLES | 2776 | 98% | 10.2,11.3[4] |
| *formyl*.MLFK.[BAT] | 884 | 96% | 11.9,13.7[2] |
| [BAT].(VPGVG)$_4$amide | 1974 | 96% | 11.9,12.8[4] |
| *formyl*.MLFK.[BAT].KKKKKamide | 1524 | 96% | 11.7,12.2[4] |
| *formyl*.MLFK.[BAT].GSGSamide | 1315 | 97% | 11.9,12.8[4] |
| *formyl*. MLFK.[BAT].E | 1013 | 99% | 12.3[4] |
| *formyl*.M.Dpg.F.[BAM] | 1354 | 98% | 13.7[4] |
| *formyl*. MLFK.[BAT].EGE | 1200 | 98% | 12.1[4] |

| Peptide Reagents | FABMS $MH^+$ | Radiochemical Yield | HPLC $R_t$(min) |
|---|---|---|---|
| (*formyl*.MLFK.[BAT].GGC$_{Acm}$GC$_{Acm}$GGCamide)$_2$BSME | 3477 | 99% | 11.9,12.4[4] |
| (*Ac*.CC$_{Acm}$GC$_{Acm}$PLYKKIIKKLLES)$_2$BSME | 4483 | 98% | 11.6[4] |
| [DTPA]C$_{Acm}$GC$_{Acm}$PLYKKIIKKLLES | 2468 | 91% | 11.4-14.0[4] |
| (*Ac*.CKKC$_{Acm}$GC$_{Acm}$PLYKKIIKKLLES)$_2$BSME | | | |
| (*Ac*.CGC$_{Acm}$GC$_{Acm}$GGPLYKKIIKKLLES)$_2$BSME | 4825* | 99% | 16.2[4] |
| (*formyl*.MLFK.GGC$_{Acm}$GC$_{Acm}$GGCamide)$_2$BSME | 2839* | | |
| [BAT].(VGVAPG)$_3$amide | 1778 | 98% | 11.4[4] |
| *Ac*.C$_{Acm}$GC$_{Acm}$QAPLYKKIILKKLLES | 2220 | 100% | 16.6[5] |
| [BAT].KKLLKKLYKKIIKKLLES | 2533 | 99% | Bound[4] |

Ac = acetyl; other abbreviations as in "Leukocyte Binding Peptides" Table above

* ESMS data (M); ** FABMS data ($MNa^+$)

EXAMPLE 3

**Scintigraphic Imaging and Biodistribution of Tc-99m Labeled Peptides**

In order to demonstrate the effectiveness of Tc-99m labeled peptide reagents as provided above, New Zealand

white rabbits were innoculated intramuscularly in the left calf with a potent stain of *E. coli*. After 24 h, the animals were sedated by i.m. injection of ketamine and xylazine, and then injected i.v. with Tc-99m labeled peptide (≤ 150µg, 2-10 mCi). The animals were positioned supine in the field of view of a gamma camera (LEAP collimator/ photopeaked for Tc-99m) and imaged over the first hour post-injection, and then at approximately 1h intervals over the next three hours post injection. Animals were allowed to recover between image acquisitions and reanesthetized as needed.

Upon completion of the final imaging, each animal was sacrificed by overdose of phenobarbital i.v. and dissected to obtain samples of blood and of infected and control muscle tissue. The tissue samples were weighed, and along with a standard amount of the injected dose, were counted using a gamma counter, and the percent injected dose (per gram of tissue) remaining in the tissues was determined. Ratios of percent of injected dose per gram of infected *versus* non-infected muscle tissue, and of infected muscle tissue *versus* blood, were calculated for each peptide. These results are presented in the following Table. Scintiphotos of whole body and leg images of a rabbit injected with a Tc-99m labeled reagent of the invention, having the formula

$$acetylKKKKKC_{Acm}GC_{Acm}GGPLYKKIIKKLLES$$

are presented in Figure 1.

| Peptide Reagents | Infected Muscle (%ID/g) | Control Muscle (%ID/g) | Ratio of Infected/ Control | Blood (%ID/g) | Ratio of Infected/ Blood |
|---|---|---|---|---|---|
| *Ac*.$C_{Acm}GC_{Acm}$GGG(VPGVG)$_4$amide | 0.0306 | 0.0077 | 3.97 | 0.049 | 0.63 |
| *Ac*.CGGGPLYKKIIKKLLES | 0.0235 | 0.0050 | 4.70 | 0.032 | 0.74 |
| *formyl*.MLFK.[BAT] amide | 0.0215 | 0.0028 | 7.68 | 0.006 | 3.58 |
| *p*Glu.GVNDNEEGFFSAR$C_{Acm}GC_{Acm}$amide | 0.0165 | 0.0029 | 5.68 | 0.024 | 0.68 |
| *formyl*.M.Dpg.F.[BAM] | 0.0106 | 0.0007 | 15.14 | 0.003 | 3.53 |
| PicGC(VGVAPG)$_3$amide | 0.0106 | 0.0019 | 5.58 | 0.015 | 0.69 |
| (*Ac*.C$C_{Acm}GC_{Acm}$PLYKKIIKKLLES)$_2$BSME | 0.0082 | 0.0011 | 7.45 | 0.010 | 0.84 |
| $C_{Acm}GC_{Acm}$(VGVAPG)$_3$amide | 0.0067 | 0.0017 | 3.94 | 0.011 | 1.69 |
| $C_{Acm}GC_{Acm}$TKPR | 0.0060 | 0.0025 | 2.40 | 0.003 | 2.07 |
| *Ac*.KKKKK$C_{Acm}GC_{Acm}$GGPLYKKIIKKLLES | 0.0061 | 0.0019 | 5.60 | 0.006 | 0.93 |
| *formyl*.Thp.LF.[BAM] | 0.0048 | 0.0010 | 4.80 | 0.006 | 0.83 |
| [BAT].(VPGVG)$_4$amide | 0.0032 | 0.0006 | 5.33 | 0.002 | 1.68 |
| [BAT].GGPLYKKIIKKLLES | 0.0021 | 0.0003 | 7.00 | 0.004 | 0.50 |

(%ID/g) = percent injected dose per gram tissue; other abbreviations are as in the previous Tables.

It should be understood that the foregoing disclosure emphasizes certain specific embodiments of the invention and that all modifications or alternatives equivalent thereto are within the spirit and scope of the invention as set forth in the appended claims.

## Claims

1. A reagent for preparing a scintigraphic imaging agent for imaging sites of inflammation within a mammalian body, comprising a specific binding peptide that specifically binds to leukocytes, covalently linked to a technetium-99m radiolabel-binding moiety, wherein the technetium-99m radiolabel-binding moiety has a formula selected from :

I.

Cp(aa)Cp

wherein Cp is a protected cysteine and (aa) is an amino acid;

II.

$$A\text{-}CZ(B)\text{-}[C(R^1R^2)]_n\text{-}X$$

wherein

A is H, HOOC, $H_2NOC$, (peptide)-NHOC, (peptide)-OOC or $R^4$;

B is H, SH, $-NHR^3$, $-N(R^3)$-(peptide), or $R^4$;

X is H, SH, $-NHR_3$, $-N(R^3)$-(peptide) or $R^4$;

Z is H or $R^4$;

$R^1$, $R^2$, $R^3$ and $R^4$ are independently H or lower straight or branched chain or cyclic alkyl;

n is 0, 1 or 2;

and

where B is $-NHR^3$ or $-N(R^3)$-(peptide), X is SH, and n is 1 or 2;

where X is $-NHR^3$ or $-N(R^3)$-(peptide), B is SH, and n is 1 or 2;

where B is H or $R^4$, A is HOOC, $H_2NOC$, (peptide)-NHOC or (peptide)-OOC,

X is SH, and n is 0 or 1;

where A is H or $R^4$, then where B is SH, X is $-NHR^3$ or $-N(R^3)$-(peptide) and where X is SH, B is $-NHR^3$ or $-N(R^3)$-(peptide);

where X is H or $R^4$, A is HOOC, $H_2$ NOC, (peptide)-NHOC or (peptide)-OOC, and B is SH;

where Z is methyl, X is methyl, A is HOOC, $H_2$ NOC, (peptide)-NHOC, or (peptide)-OOC, B is SH and n is 0;

where B is SH and X is SH, n is not 0;

and wherein the thiol moiety is in the reduced form;

III.

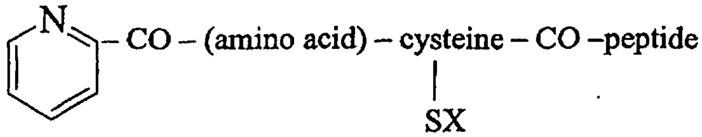

IV.

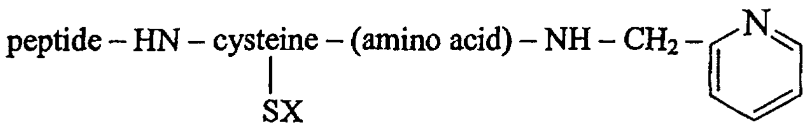

wherein

X = H or a protecting group;

(amino acid) = any amino acid;

V.

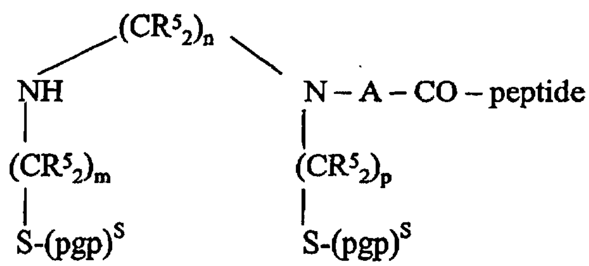

wherein

each $R^5$ is independently H, $CH_3$ or $C_2H_5$;

each $(pgp)^s$ is independently a thiol protecting group or H;

m, n and p are independently 2 or 3;

A = linear or cyclic lower alkyl, aryl, heterocyclyl, combinations or substituted derivatives thereof;

VI.

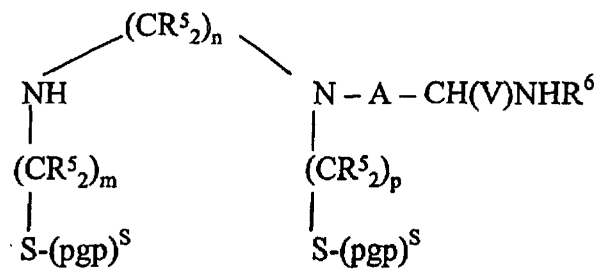

wherein

each $R^5$ is independently H, lower alkyl having 1 to 6 carbon atoms, phenyl, or phenyl substituted with lower alkyl or lower alkoxy;

m, n and p are independently 1 or 2;

A = linear or cyclic lower alkyl, aryl, heterocyclyl, or combinations or substituted derivatives thereof;

V = H or -CO-peptide;

$R^6$ = H or peptide;

and wherein when V = H, $R^6$ = peptide and when $R^6$ = H, V = -CO-peptide; and wherein the radiolabel-binding moiety forms a complex with a radioisotope and the complex is electrically neutral;

with the proviso that when the technetium-99m radiolabel-binding moiety has the formula Cp(aa)Cp, wherein Cp is a protected cysteine and (aa) is an amino acid, the specific binding peptide does not contain the amino acid sequence

- PLYKKIIKKLLES;
- *formyl*.Nle.LF.Nle.YK;
- *formyl*.MIFL;
- *formyl*.MLFK;
- *formyl*.MLFI;
- *formyl*.MFIL;
- *formyl*.MFLI;
- *formyl*.MLIF;
- *formyl*.MILF;
- *formyl*.MLF;
- VGVAPG;
- VGVAPGVGVAPGVGVAPG;
- VPGVGVPGVGVPGVGVPGVG;
- TKPR.

**2.** A reagent of claim 1, wherein the specific binding peptide and the technetium-99m radiolabel-binding moiety are covalently linked through one or more amino acids.

**3.** A reagent of claim 1, wherein the peptide is selected from the group consisting of platelet factor 4, a derivative of platelet factor 4, *formyl*MLF, *formyl*Nle.LF.Nle, a derivative of *formyl*MLF, a derivative of *formyl*Nle.LF.Nle, tuftsin, a derivative of tuftsin, fibrinopeptide B, fibrinogen B β-chain, a derivative of fibrinopeptide B, and a derivative of fibrinogen B β-chain.

**4.** A reagent of claim 3 having the formula selected from the group consisting of:

Pic.G$C_{Acm}$PLYKKIIKKLLES;
*acetyl*.CGGGPLYKKIIKKLLES;
[BAT].GGPLYKKIIKKLLES;
*acetyl*.KKKKK$C_{Acm}$ G$C_{Acm}$GGPLYKKIIKKLLES;
[DTPA].$C_{Acm}$G$C_{Acm}$PLYKKIIKKLLES;
[BAT].KKLLKKLYKKIIKKLLES;
*Ac*.$C_{Acm}$G$C_{Acm}$QAPLYKKIIKKLLES;

*formyl*.MLF$C_{Acm}$GPica;
*formyl*.MLFK.[BAT].amide;
*formyl*.Thp.LF.[BAM];
*formyl*.MLFK.[BAT];
*formyl*.MLFK.[BAT].KKKKK.amide;
*formyl*.MLFK.[BAT].GSGS.amide;
*formyl*.MLFK.[BAT].E;
*formyl*.M.Dpg.F.[BAM];
*formyl*.MLFK.[BAT].EGE;

$C_{Acm}$G$C_{Acm}$$(VGVAPG)_3$ amide;
Pic.G$C_{Acm}$$(VGVAPG)_3$ amide;
Pic.G$C_{Acm}$$(VPGVG)_4$ amide;
$(VPGVG)_4$GGG$C_{Acm}$G$C_{Acm}$ amide;
$(VGVAPG)_3$GGG$C_{Acm}$G$C_{Acm}$ amide;
*acetyl*.$C_{Acm}$G$C_{Acm}$GGG$(VPGVG)_4$ amide;
*acetyl*.$C_{Acm}$G$C_{Acm}$.Aca.$(VPGVG)_4$ amide;
Pic.GC$(VGVAPG)_3$ amide;
[BAT].$(VGVAPG)_3$ amide;
[BAT].$(VPGVG)_4$ amide;
*p*Glu.GVNDNEEGFFSAR$C_{Acm}$G$C_{Acm}$amide;
*p*Glu.GVNDNEEGFFSARGGC.amide;
Pic.G$C_{Acm}$GHRPLDKKREEAPSLRPAPPPISGGGYR;
[BAT].GHRPLDKKREEAPSLRPAPPPISGGGYR.amide .

**5.** A reagent of claim 1 comprising

a) a multiplicity of leukocyte-binding peptides;

b) a multiplicity of technetium-99m radiolabel-binding moieties; and

c) a polyvalent linker moiety;

wherein the polyvalent linker moiety is covalently linked to the multiplicity of the peptides, the technetium-99m radiolabel-binding moieties, or both, to comprise a multimeric polyvalent reagent, wherein the molecular weight of the multimeric polyvalent reagent is less than about 20,000 daltons; preferably wherein the polyvalent linker is selected from the group consisting of *bis*-succinimidylmethylether, 4-(2,2-dimethylacetyl)benzoic acid, *tris*(succinimidylethyl)amine, a derivative of *bis*-succinimidyl-methylether, a derivative of 4-(2,2-dimethylacetyl)benzoic acid, and a derivative of *tris*(succinimidylethyl)amine.

**6.** The reagent of claim 5, having the formula selected from the group consisting of:

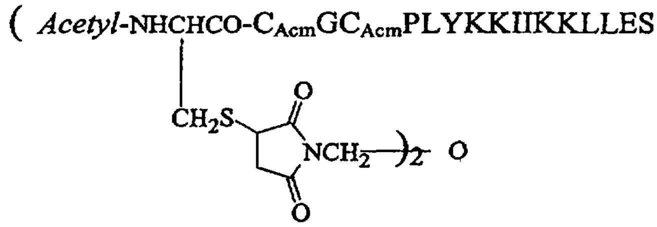

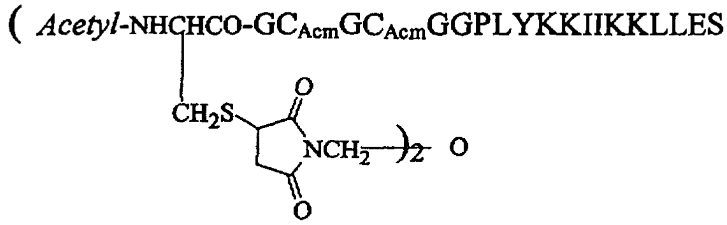

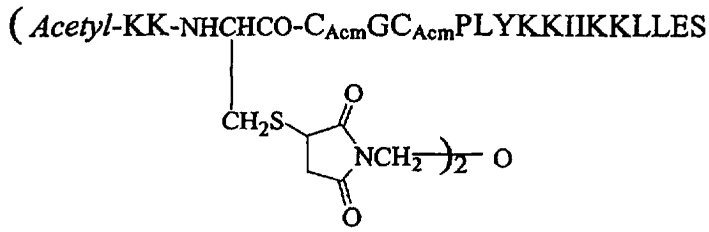

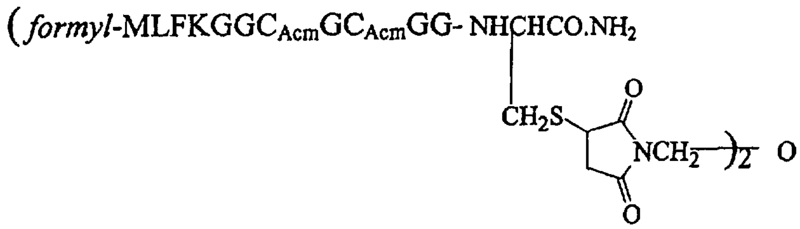

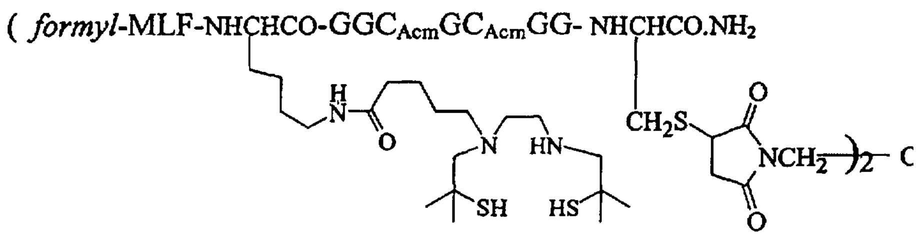

**7.** A scintigraphic imaging agent comprising the reagent of any one of claims 1 to 6 radiolabeled with technetium-99m.

**8.** A complex formed by either, (a) reacting a reagent of any one of claims 1 to 6 with technetium-99m in the presence of a reducing agent, preferably a reducing agent selected from the group consisting of a dithionite ion, a stannous ion, and a ferrous ion, or (b) labeling the reagent of any one of claims 1 to 6 with technetium-99m by ligand exchange of a prereduced technetium-99m complex.

**9.** A kit for preparing a radiopharmaceutical preparation, said kit comprising a sealed vial containing a predetermined quantity of a reagent of any one of claims 1 to 6 and a sufficient amount of reducing agent to label the reagent with technetium-99m.

**10.** A process of preparing a reagent of claim 1, wherein the reagent is chemically synthesized in vitro, preferably wherein the specific binding peptide is synthesized by solid phase peptide synthesis.

**11.** The process of claim 10, wherein the radiolabel-binding moiety is covalently linked to the specific binding peptide during in vitro chemical synthesis; preferably wherein the radiolabel-binding moiety is covalently linked to the specific binding peptide during solid phase peptide synthesis.

**12.** A method for labeling a reagent of claim 1, comprising the step of reacting the reagent with technetium-99m in the presence of a reducing agent, preferably wherein the reducing agent is selected from the group consisting of a dithionite ion, a stannous ion, and a ferrous ion.

**13.** The complex of claim 8 for use for imaging a site of inflammation within a mammalian body.

**14.** Use of the reagent of any one of claims 1 to 6 in the manufacture of a diagnostic medicament for imaging a site of inflammation within a mammalian body.

**Patentansprüche**

**1.** Reagens zur Herstellung eines szintigraphischen bilderzeugenden Mittels zur Abbildung von Entzündungsherden im Körper eines Säugetieres, umfassend ein spezifisch bindendes Peptid, das spezifisch an Leukozyten bindet und kovalent mit einer einen radioaktiven Technetium-99m-Marker bindenden Einheit verbunden ist, wobei die den radioaktiven Technetium-99m-Marker bindende Einheit eine aus:

I.

Cp(aa)Cp

wobei Cp für ein geschütztes Cystein und (aa) für eine Aminosäure steht;

II.

$$A\text{-}CZ(B)\text{-}[C(R^1R^2)]_n\text{-}X$$

wobei

A für H, HOOC, $H_2NOC$, (Peptid)-NHOC, (Peptid)-OOC oder $R^4$ steht;

B für H, SH, $-NHR^3$, $-N(R^3)$-(Peptid) oder $R^4$ steht;

X für H, SH, $-NHR^3$, $-N(R^3)$-(Peptid) oder $R^4$ steht;

Z für H oder $R^4$ steht;

$R^1$, $R^2$, $R^3$ und $R^4$ unabhängig voneinander für H oder niederes lineares oder verzweigtes oder cyclisches Alkyl stehen;

n für 0, 1 oder 2 steht;

und, wenn B für $-NHR^3$ oder $-N(R^3)$-(Peptid) steht, X für SH und n für 1 oder 2 steht;

wenn X für $-NHR^3$ oder $-N(R^3)$-(Peptid) steht, B für SH und n für 1 oder 2 steht;

wenn B für H oder $R^4$ steht, A für HOOC, $H_2NOC$, (Peptid)-NHOC oder (Peptid)-OOC, X für SH und n für 0 oder 1 steht;

wenn A für H oder $R^4$ steht, im Fall, daß B für SH steht, X für $-NHR^3$ oder $-N(R^3)$-(Peptid) steht, und, im Fall, daß X für SH steht, B für $-NHR^3$ oder $-N(R^3)$-(Peptid) steht;

wenn X für H oder $R^4$ steht, A für HOOC, $H_2NOC$, (Peptid)-NHOC oder (Peptid)-OOC und B für SH steht;

wenn Z für Methyl steht, X für Methyl, A für HOOC, $H_2NOC$, (Peptid)-NHOC oder (Peptid)-OOC, B für SH und n für 0 steht;

wenn B für SH und X für SH steht, n nicht für 0 steht;

und wobei die Thioleinheit in reduzierter Form vorliegt;

III.

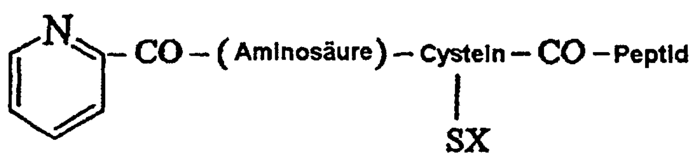

IV.

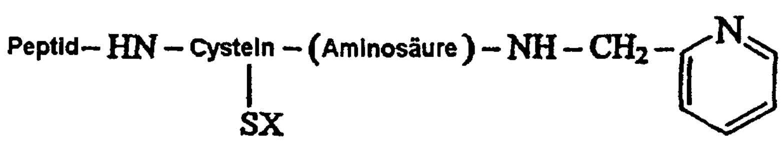

wobei

X = H oder eine Schutzgruppe;

(Aminosäure) = eine beliebige Aminosäure;

V.

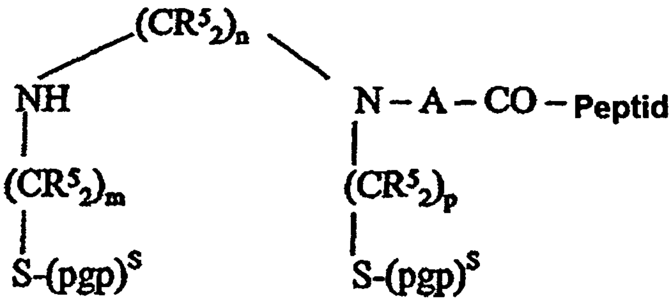

wobei die Reste $R^5$ jeweils unabhängig voneinander für H, $CH_3$ oder $C_2H_5$ stehen;

die Reste $(pgp)^S$ jeweils unabhängig voneinander für eine Thiol-Schutzgruppe oder H stehen;

m, n und p unabhängig voneinander für 2 oder 3 stehen;

A = lineares oder cyclisches niederes Alkyl, Aryl, Heterocyclyl, Kombinationen oder substituierte Derivate davon;

VI.

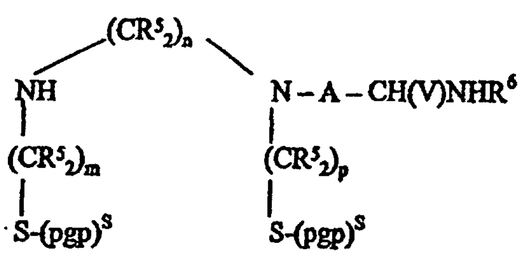

wobei die Reste $R^5$ jeweils unabhängig voneinander für H, niederes Alkyl mit 1 bis 6 Kohlenstoffatomen, Phenyl oder durch niederes Alkyl oder niederes Alkoxy substituiertes Phenyl stehen;

m, n und p unabhängig voneinander für 1 oder 2 stehen;

A = lineares oder cyclisches niederes Alkyl, Aryl, Heterocyclyl, oder Kombinationen oder substituierte Derivate davon;

V = H oder -CO-Peptid;

$R^6$ = H oder Peptid;

und wobei, wenn V = H, $R^6$ = Peptid und, wenn $R^6$ = H, V = -CO-Peptid, und wobei die den radioaktiven Marker bindende Einheit einen Komplex mit einem Radioisotop bildet und der Komplex elektrisch neutral ist;

ausgewählte Formel aufweist,
mit der Maßgabe, daß, wenn die den radioaktiven Technetium-99m-Marker bindende Einheit die Formel Cp(aa) Cp aufweist, wobei Cp für geschütztes Cystein und (aa) für eine Aminosäure steht, das spezifisch bindende Peptid nicht die Aminosäuresequenz

- PLYKKIIKKLLES;
- formyl.Nle.LF.Nle.YK;
- *formyl*.MIFL;
- *formyl*.MLFK;
- *formyl*.MLFI;
- *formyl*.MFIL;
- *formyl*.MFLI;
- *formyl*.MLIF;
- *formyl*.MILF;
- *formyl*.MLF;
- VGVAPG;
- VGVAPGVGVAPGVGVAPG;
- VPGVGVPGVGVPGVGVPGVG;
- TKPR;

enthält.

**2.** Reagens nach Anspruch 1, wobei das spezifisch bindende Peptid und die den radioaktiven Technetium-99m-Marker bindende Einheit über eine oder mehrere Aminosäuren kovalent verbunden sind.

**3.** Reagens nach Anspruch 1, wobei das Peptid aus der aus Plättchenfaktor 4, einem Derivat von Plättchenfaktor 4, *formyl*MLF, *formyl*Nle.LF.Nle, einem Derivat von *formyl*MLF, einem Derivat von *formyl*Nle.LF.Nle, Tuftsin, einem Derivat von Tuftsin, Fibrinopeptid B, der β-Kette von Fibrinogen B, einem Derivat von Fibrinopeptid B und einem Derivat der β-Kette von Fibrinogen B bestehenden Gruppe ausgewählt ist.

**4.** Reagens nach Anspruch 3 mit einer Formel ausgewählt aus der aus:

Pic.GC$_{Acm}$PLYKKIIKKLLES;
*acetyl*.CGGGPLYKKIIKKLLES;
[BAT].GGPLYKKIIKKLLES;
*acetyl*.KKKKKC$_{Acm}$GC$_{Acm}$GGPLYKKIIKKLLES;
[DTPA].C$_{Acm}$GC$_{Acm}$PLYKKIIKKLLES;
[BAT].KKLLKKLYKKIIKKLLES;
*Ac*.C$_{Acm}$GC$_{Acm}$QAPLYKKIIKKLLES;

*formyl*.MLFC$_{Acm}$GPica;
*formyl*.MLFK.[BAT].Amid;
*formyl*.Thp.LF.[BAM];
*formyl*.MLFK.[BAT];
*formyl*.MLFK.[BAT].KKKKK.Amid;
*formyl*.MLFK.[BAT].GSGS.Amid;
*formyl*.MLFK.[BAT].E;
*formyl*.M.Dpg.F.[BAM];
*formyl*.MLFK.[BAT].EGE;

C$_{Acm}$GC$_{Acm}$(VGVAPG)$_3$Amid;
Pic.GC$_{Acm}$(VGVAPG)$_3$Amid;
Pic.GC$_{Acm}$(VPGVG)$_4$Amid;
(VPGVG)$_4$GGGC$_{Acm}$GC$_{Acm}$ Amid;
(VGVAPG)$_3$GGGC$_{Acm}$GC$_{Acm}$ Amid;
*acetyl*.C$_{Acm}$GC$_{Acm}$GGG(VPGVG)$_4$ Amid;
*acetyl*.C$_{Acm}$GC$_{Acm}$.Aca.(VPGVG)$_4$ Amid;
Pic.GC(VGVAPG)$_3$ Amid;
[BAT].(VGVAPG)$_3$ Amid;
[BAT].(VPGVG)$_4$ Amid;

*p*Glu.GVNDNEEGFFSARC$_{Acm}$GC$_{Acm}$Amid;
*p*Glu.GVNDNEEGFFSARGGC.Amid;

Pic.$GC_{Acm}$GHRPLDKKREEAPSLRPAPPPISGGGYR;
[BAT].GHRPLDKKREEAPSLRPAPPPISGGGYR.Amid.

bestehenden Gruppe.

**5.** Reagens nach Anspruch 1, enthaltend

a) eine Vielzahl von Leukozyten bindenden Peptiden;
b) eine Vielzahl von radioaktiven Technetium-99m-Marker bindenden Einheiten; und
c) eine polyvalente Verbindungseinheit;

wobei die polyvalente Verbindungseinheit kovalent mit der Vielzahl an Peptiden, den den radioaktiven Technetium-99m-Marker bindenden Einheiten oder beiden verbunden ist, so daß man ein multimeres polyvalentes Reagens erhält, wobei das Molekulargewicht des multimeren polyvalenten Reagens weniger als etwa 20000 Dalton beträgt; wobei die polyvalente Verbindungseinheit bevorzugt aus der aus Bis-succinimidylmethylether, 4-(2,2-Dimethylacetyl)benzoesäure, *Tris*(succinimidylethyl)amin, einem Derivat von *Bis*-succinimidylmethylether, einem Derivat von 4-(2,2-Dimethylacetyl)-benzoesäure und einem Derivat von *Tris*(succinimidylethyl)amin bestehenden Gruppe ausgewählt ist.

**6.** Reagens nach Anspruch 5, mit einer Formel ausgewählt aus der aus:

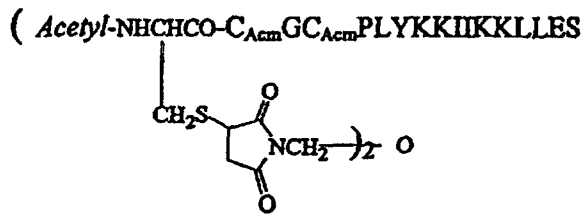

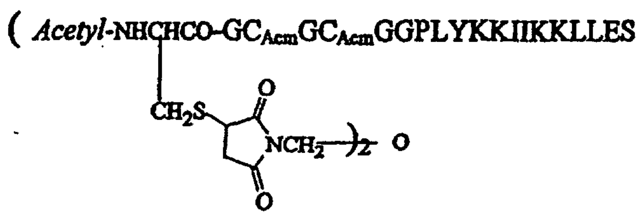

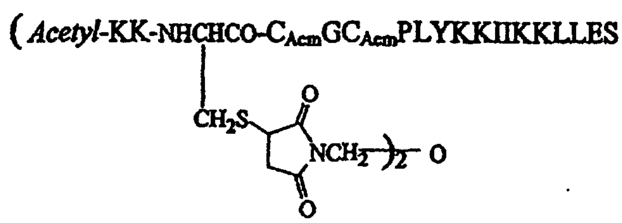

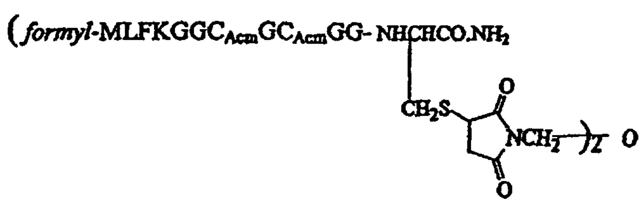

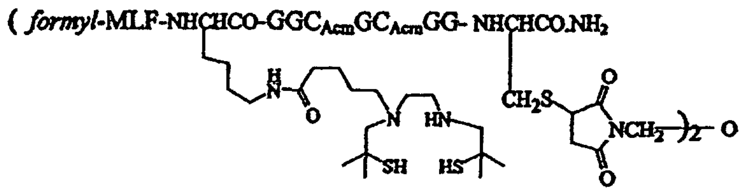

bestehenden Gruppe.

**7.** Szintigraphisches bilderzeugendes Mittel, umfassend das Reagens nach einem der Ansprüche 1 bis 6, radioaktiv mit Technetium-99m markiert.

**8.** Komplex, gebildet durch (a) Umsetzung eines Reagens nach einem der Ansprüche 1 bis 6 mit Technetium-99m in Gegenwart eines Reduktionsmittels; vorzugsweise eines aus der aus einem Dithionition, einem Zinn(II)-Ion und einem Eisen(II)-Ion bestehenden Gruppe, oder (b) Markieren des Reagens nach einem der Ansprüche 1 bis 6 mit Technetium-99m durch Ligandenaustausch eines vorreduzierten Technetium-99m-Komplexes.

**9.** Kit zur Herstellung einer radiopharmazeutischen Zubereitung, wobei das Kit ein verschlossenes Vial mit einer vorbestimmten Menge eines Reagens nach einem der Ansprüche 1 bis 6 und eine zur Markierung des Reagens mit Technetium-99m ausreichende Menge eines Reduktionsmittels enthält.

**10.** Verfahren zur Herstellung eines Reagens nach Anspruch 1, bei dem man das Reagens chemisch in vitro synthetisiert, wobei das spezifisch bindende Peptid vorzugsweise durch Festphasenpeptidsynthese synthetisiert wird.

**11.** Verfahren nach Anspruch 10, bei dem die den radioaktiven Marker bindende Einheit während der chemischen in-vitro-Synthese kovalent mit dem spezifisch bindenden Peptid verbunden wird; wobei die den radioaktiven Marker bindende Einheit vorzugsweise während einer Festphasenpeptidsynthese kovalent mit dem spezifisch bindenden Peptid verbunden wird.

**12.** Verfahren zum Markieren eines Reagens nach Anspruch 1, bei dem man das Reagens in Gegenwart eines Reduktionsmittels, das vorzugsweise aus der aus einem Dithionition, einem Zinn(II)-Ion und einem Eisen(II)-Ion bestehenden Gruppe ausgewählt ist, mit Technetium-99m umsetzt.

**13.** Komplex nach Anspruch 8 zur Verwendung bei der Abbildung von Entzündungsherden im Körper eines Säugetieres.

**14.** Verwendung der Reagens nach einem der Ansprüche 1 bis 6 zur Herstellung eines Diagnostikums für die Abbildung von Entzündungsherden im Körper eines Säugetieres.

**Revendications**

**1.** Réactif pour préparer un agent d'imagerie scintigraphique pour visualiser des sites d'inflammation à l'intérieur d'un corps de mammifère, comprenant un peptide de liaison spécifique qui se lie spécifiquement aux leucocytes et est lié de manière covalente à un fragment de liaison aux radiomarquages au technétium 99m, dans lequel le fragment de liaison aux radiomarquages au technétium 99m présente l'une des formules suivantes:

I.

Cp(aa)Cp

dans laquelle Cp est une cystéine protégée et (aa) est un acide aminé;

II.

$$A\text{-}CZ(B)\text{-}[C(R^1R^2)]_n\text{-}X$$

dans laquelle

A est H, HOOC, $H_2NOC$, (peptide)-NHOC, (peptide) -OOC ou $R^4$;

B est H, SH, $-NHR^3$, $-N(R^3)$- (peptide) ou $R^4$;

X est H, SH, $-NHR^3$, $-N(R^3)$- (peptide) ou $R^4$;

Z est H ou $R^4$;

$R^1$, $R^2$, $R^3$ et $R^4$ sont indépendamment H ou un alkyle inférieur cyclique ou à chaîne linéaire ou ramifiée;

n est 0, 1 ou 2;

et

lorsque B est $-NHR^3$ ou $-N(R^3)$-(peptide), X est SH et n est 1 ou 2;

lorsque X est $-NHR^3$ ou $-N(R^3)$-(peptide), B est SH et n est 1 ou 2;

lorsque B est H ou $R^4$, A est HOOC, $H_2NOC$, (peptide)-NHOC ou (peptide)-OOC, X est SH et n est 0 ou 1;

lorsque A est H ou $R^4$, alors lorsque B est SH, X est $-NHR^3$ ou $-N(R^3)$-(peptide) et lorsque X est SH, B est $-NHR^3$ ou $-N(R^3)$-(peptide);

lorsque X est H ou $R^4$, A est HOOC, $H_2NOC$, (peptide)-NHOC ou (peptide)-OOC et B est SH;

lorsque Z est le méthyle, X est le méthyle, A est HOOC, $H_2NOC$, (peptide)-NHOC ou (peptide)-OOC, B est SH et n est 0;

lorsque B est SH et X est SH, n est différent de 0;

et dans laquelle le fragment thiol est sous forme réduite;

III.

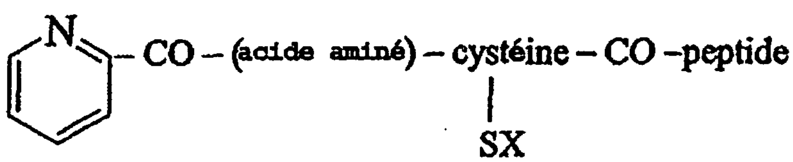

IV.

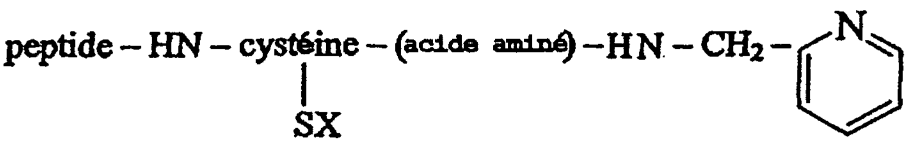

dans laquelle

X = H ou un groupe protecteur;
(acide aminé) = n'importe quel acide aminé;

V.

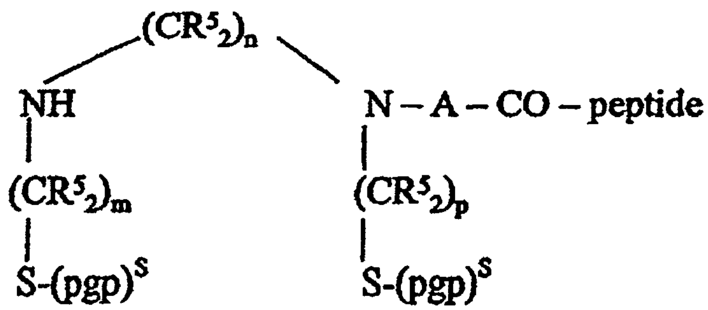

dans laquelle
chaque $R^5$ est indépendamment H, $CH_3$ ou $C_2H_5$;
chaque $(pgp)^S$ est indépendamment un groupe protecteur de thiol ou H;
m, n et p sont indépendamment 2 ou 3;
A = un alkyle inférieur linéaire ou cyclique, un aryle, un hétérocyclyle, des combinaisons ou des substitués de ceux-ci;

VI.

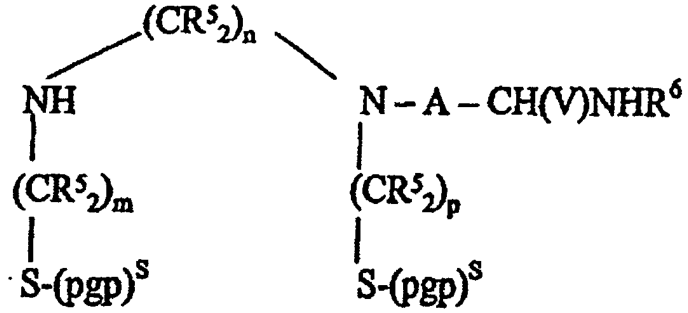

dans laquelle
chaque $R^5$ est indépendamment H, un alkyle inférieur ayant 1 à 6 atomes de carbone, le phényle ou le phényle substitué avec un alkyle inférieur ou un alcoxy inférieur;
chaque m, n et p est indépendamment 1 ou 2;

A = un alkyle inférieur linéaire ou cyclique, un aryle, un hétérocyclyle, des combinaisons ou des dérivés substitués de ceux-ci;
V = H ou -CO-peptide;
$R^6$ = H ou peptide;

et où quand V = H, $R^6$ = peptide et quand $R^6$ = H, V = -CO-peptide; et où le fragment de liaison aux radiomarquages forme un complexe avec un radio-isotope et le complexe est électriquement neutre ;

à condition que, quand le fragment de liaison aux radiomarquages au technétium 99m est de formule Cp (aa)Cp, dans laquelle Cp est une cystéine protégée et (aa) est un acide aminé, le peptide de liaison spécifique ne contienne pas la séquence d'acide aminé

- PLYKKIIKKLLES ;
- *formyl*.Nle.LF.Nle.YK ;
- *formyl*.MIFL ;
- *formyl*.MLFK ;
- *formyl*.MLFI ;
- *formyl*.MFIL ;
- *formyl*.MFLI ;
- *formyl*.MLIF ;
- *formyl*.MILF ;
- *formyl*.MLF ;
- VGVAPG ;
- VGVAPGVGVAPGVGVAPG ;
- VPGVGVPGVGVPGVGVPGVG ;
- TKPR.

2. Réactif selon la revendication 1, dans lequel le peptide de liaison spécifique et le fragment de liaison aux radiomarquages au technétium 99m sont liés de manière covalente par un ou plusieurs acides aminés.

3. Réactif selon la revendication 1, dans lequel le peptide est choisi parmi le groupe constitué du facteur plaquettaire 4, d'un dérivé du facteur plaquettaire 4, de *formyl*MLF, de *formyl*Nle.LF.Nle, d'un dérivé de *formyl*MLF, d'un dérivé de formylNle.LF.Nle, de tuftsine, d'un dérivé de tuftsine, du fibrinopeptide B, de la chaîne β du fibrinogène B, d'un dérivé du fibrinopeptide B et d'un dérivé de la chaîne β du fibrinogène B.

4. Réactif selon la revendication 3 ayant la formule choisie parmi le groupe constitué de :

Pic.GC$_{Acm}$PLYKKIIKKLLES;
*acétyl*.CGGGPLYKKIIKKLLES;
[BAT].GGPLYKKIIKKLLES;
*acétyl*.KKKKKC$_{Acm}$GC$_{Acm}$GGPLYKKIIKKLLES;
[DTPA].C$_{Acm}$GC$_{Acm}$PLYKKIIKKLLES;
[BAT].KKLLKKLYKKIIKKLLES;
Ac.C$_{Acm}$GC$_{Acm}$QAPLYKKIIKKLLES;

*formyl*.MLFC$_{Acm}$GPica;
*formyl*.MLFK.[BAT].amide;
*formyl*.Thp.LF.[BAM];
*formyl*.MLFK.[BAT];
*formyl*.MLFK.[BAT].KKKKK.amide;
*formyl*.MLFK.[BAT].GSGS.amide;
*formyl*.MLFK.[BAT].E;
*formyl*.M.Dpg.F.[BAM];
*formyl*.MLFK.[BAT].EGE;

C$_{Acm}$GC$_{Acm}$(VGVAPG)$_3$amide;
Pic.GC$_{Acm}$(VGVAPG)$_3$amide;
Pic.GC$_{Acm}$(VPGVG)$_4$amide;
(VPGVG)$_4$GGGC$_{Acm}$GC$_{Acm}$amide;

(VGVAPG)$_3$GGGC$_{Acm}$GC$_{Acm}$amide;
*acétyl*.C$_{Acm}$GC$_{Acm}$GGG(VPGVG)$_4$amide;
*acétyl*.C$_{Acm}$GC$_{Acm}$.Aca. (VPGVG)$_4$amide;
Pic.GC(VGVAPG)$_3$amide;
[BAT].(VGVAPG)$_3$amide;
[BAT].(VPGVG)$_4$amide;
*p*Glu.GVNDNEEGFFSARC$_{Acm}$GC$_{Acm}$amide;
*p*Glu.GVNDNEEGFFSARGGC.amide ;
Pic.GC$_{Acm}$GHRPLDKKREEAPSLRPAPPPISGGGYR;
[BAT].GHRPLDKKREEAPSLRPAPPPISGGGYR.amide.

**5.** Réactif selon la revendication 1 comprenant

a) une multiplicité de peptides de liaison aux leucocytes;
b) une multiplicité de fragments de liaison aux radiomarquages au technétium 99m; et
c) un fragment de lieur polyvalent;

dans lequel le fragment de lieur polyvalent est lié de manière covalente à la multiplicité des peptides, aux fragments de liaison aux radiomarquages au technétium 99m, ou aux deux, pour comprendre un réactif polyvalent multimère, dans lequel le poids moléculaire du réactif polyvalent multimère est inférieur à environ 20 000 daltons; de préférence dans lequel le lieur polyvalent est choisi parmi le groupe constitué du bis-succinimidylméthyléther, de l'acide 4-(2,2-diméthylacétyl)benzoïque, de la tris(succinimidyléthyl)amine, d'un dérivé de bis-succinimidylméthyléther, d'un dérivé de l'acide 4-(2,2-diméthylacétyl)benzoïque et d'un dérivé de la tris(succinimidyléthyl)amine.

**6.** Réactif selon la revendication 5, ayant la formule choisie parmi le groupe constitué de:

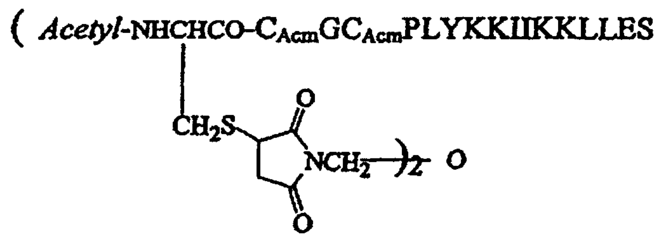

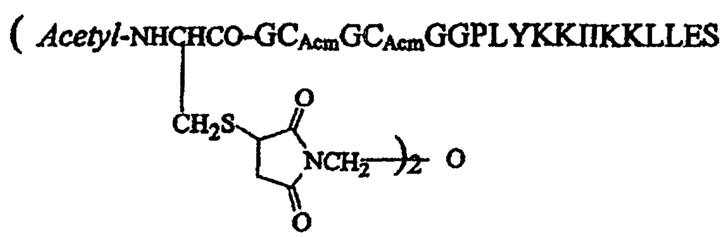

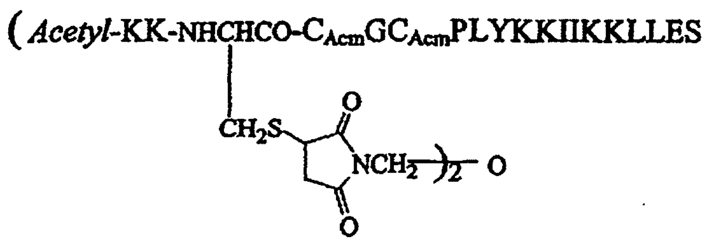

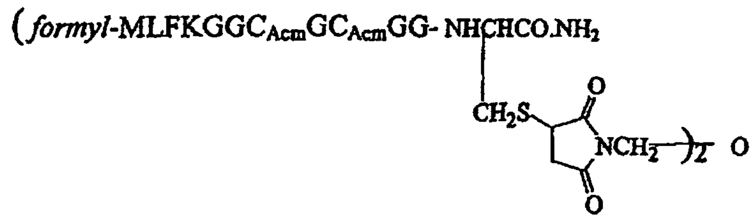

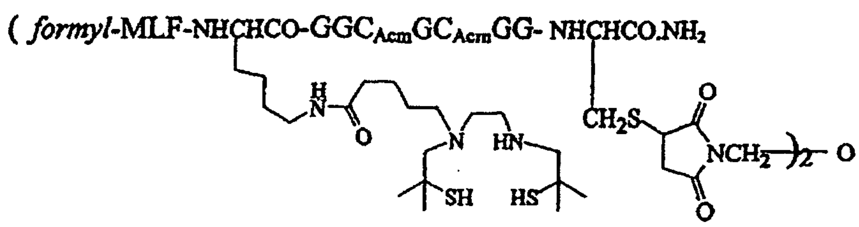

**7.** Agent d'imagerie scintigraphique comprenant le réactif selon l'une quelconque des revendications 1 à 6 radiomarqué au technétium 99m.

**8.** Complexe formé soit par (a) la réaction d'un réactif selon l'une quelconque des revendications 1 à 6 avec le technétium 99m en présence d'un agent réducteur, de préférence un agent réducteur choisi parmi le groupe constitué d'un ion dithionite, d'un ion stanneux et d'un ion ferreux, soit par (b) le marquage du réactif selon l'une quelconque des revendications 1 à 6 au technétium 99m par échange de ligand d'un complexe de technétium 99m préréduit.

**9.** Trousse pour préparer une préparation radiopharmaceutique, ladite trousse comprenant un flacon scellé contenant une quantité prédéterminée d'un réactif selon l'une quelconque des revendications 1 à 6 et une quantité suffisante d'agent réducteur pour marquer le réactif au technétium 99m.

**10.** Procédé de préparation d'un réactif selon la revendication 1, dans lequel le réactif est chimiquement synthétisé in vitro, de préférence dans lequel le peptide de liaison spécifique est synthétisé par synthèse peptidique en phase solide.

**11.** Procédé selon la revendication 10, dans lequel le fragment de liaison aux radiomarquages est lié de manière covalente au peptide de liaison spécifique durant la synthèse chimique in vitro; de préférence dans lequel le fragment de liaison aux radiomarquages est lié de manière covalente au peptide de liaison spécifique durant la synthèse peptidique en phase solide.

**12.** Procédé de marquage d'un réactif selon la revendication 1, comprenant l'étape de réaction du réactif avec le technétium 99m en présence d'un agent réducteur, de préférence dans lequel l'agent réducteur est choisi parmi le groupe constitué d'un ion dithionite, d'un ion stanneux et d'un ion ferreux.

**13.** Complexe selon la revendication 8 dont l'utilisation vise à visualiser un site d'inflammation à l'intérieur d'un corps de mammifère.

**14.** Utilisation du réactif selon l'une quelconque des revendications 1 à 6 dans la fabrication d'un médicament diagnostique pour visualiser un site d'inflammation à l'intérieur d'un corps de mammifère.

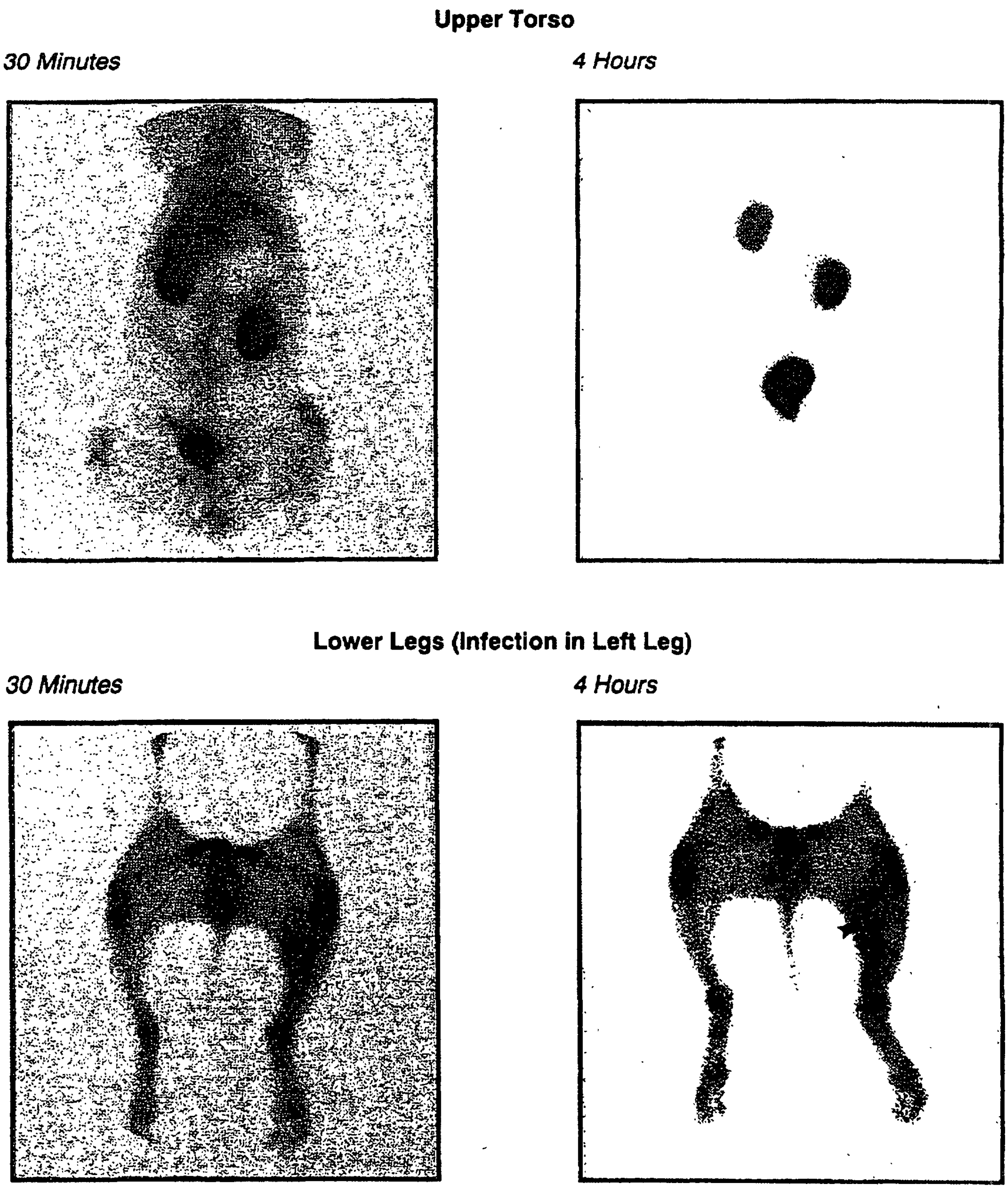
Upper Torso
30 Minutes
4 Hours
Lower Legs (Infection in Left Leg)
30 Minutes
4 Hours

FIGURE 1